# EUROPÄISCHE PATENTSCHRIFT

(11) **EP 1 930 043 B1**
(45) Veröffentlichungstag und Bekanntmachung des Hinweises auf die Patenterteilung: **25.04.2018**
(21) Anmeldenummer: 07023457.0
(22) Anmeldetag: 04.12.2007
(51) Int. Cl.: A61B 5/091, A61M 16/10, A61M 16/00, A61M 16/12, A61M 16/20

(54) **Verfahren zur Ermittlung der Konzentration von Sauerstoff in einem Atemgasgemisch**
Method for determining oxygen concentration in a breathing gas mixture
Procédé pour déterminer la concentration d'oxygène dans un mélange gazeux pour la respiration

(30) Priorität: 05.12.2006 DE 102006057622
(43) Veröffentlichungstag der Anmeldung: 11.06.2008
(73) Patentinhaber: Löwenstein Medical Technology S.A., 2557 Luxembourg (LU)
(72) Erfinder: Dietz, Florian, 23554 Lübeck (DE); Adamelz, Benjamin, 22525 Hamburg (DE)
(74) Vertreter: Klickow & Wetzel PartGmbB

(56) Entgegenhaltungen:
- EP-A1- 0 659 445
- EP-A1- 1 334 741
- EP-A2- 0 916 358
- WO-A1-98/31282
- DE-C2- 19 626 924
- US-A- 4 637 384

## Beschreibung

Die Erfindung wird im unabhängigen Anspruch 1 definiert. In vielen Fällen reicht die Versorgung eines Patienten mittels einer Atemmaske nur mit Umgebungsluft nicht aus und es muss Sauerstoff beigemischt werden. Die Konzentration des Sauerstoffs variiert hierbei von dem normalen in der Umgebungsluft enthaltenen Anteil bis zu einem Anteil von 60%. Es sind Atemmaskensysteme bekannt und erhältlich, die über eine Atemmaske, die an einen Schlauch angeschlossen ist, ein Gasgemisch aus Sauerstoff und Umgebungsluft mit vorgegebener Sauerstoffkonzentration zu den Atemorganen eines Patienten zuführen.

Der Sauerstoff wird hierbei grundsätzlich auf zwei verschiedene Arten mit der Umgebungsluft vermischt. Nach einer der beiden Arten der Gasgemischzubereitung wird der Sauerstoff mit der Umgebungsluft in einer Vorkammer eines elektromechanischen Gerätes zusammengefügt und anschließend über einen Schlauch zur Atemmaske und somit zu den Atemorganen des Patienten mittels einer Gasquelle unter Druckerzeugung zugeführt. Diese Art der Luftgemischzubereitung wird vor allem in den Intensiv-Beatmungsgeräten eingesetzt. Als Gasquelle sind erfindungsgemäß alternativ Luftpumpen, Pumpen, Gebläse und Druckgasbehälter zu verstehen oder weitere Quellen für Beatmungsgase.

Nach einer zweiten Art der Gasgemischzubereitung wird der Sauerstoff über eine eigene Schlauchzuleitung, die vorzugsweise als ein dünnes flexibles Rohr innen in dem Luftzuführungsschlauch der Atemmaske verlegt ist, bis in den so genannten Totraum der Atemmaske, d.h. den sich unterhalb der Atemwegeglocke einer Atemmaske und den von ihr abgedeckten Gesichtspartien des Patienten gebildeten Raum, geführt, wo sich der austretende Sauerstoff mit der Luft vermischt.

Diese Art der Luftgemischzubereitung wird vorwiegend in den Heimbeatmungssystemen eingesetzt.

Eine Verlegung des Sauerstoffschlauchs außerhalb des Luft zuführenden Schlauchs bis zur Atemmaske führt zum gleichen Ergebnis. Der Sauerstoff liegt gewöhnlich komprimiert in einer Druckflasche vor und strömt daher unter eigenem Druck ins System. Die Umgebungsluft wird zumeist von einer Gasquelle angesaugt und unter Druck weiter zu dem Vermischungssystem geleitet.

Ein bestimmter Patient muss nach ärztlicher Anweisung eine bestimmte Konzentration des Sauerstoffs in seinem Atemgasgemisch erhalten, und zwar auch ohne Aufsicht über mehrere Stunden oder gar Tage. Für beide oben beschriebenen Arten der Atemgasgemisch-Zubereitung besteht hierbei ein grundsätzliches Problem, das sich durch die Ausatmung der verbrauchten Luft durch den Patienten einstellt.

Die mit CO2-Gas angereicherte ausgeatmete Luft wird von dem Patienten in den oben erwähnten Totraum der Atemmaske gegen den dort herrschenden Überdruck ausgeatmet und sie kann gewöhnlich über die hierfür vorgesehenen Öffnungen oder über ein spezielles Diffusionsfilter entweichen. Hierbei kann das in dem zuführenden Luftschlauch stehendes Gasgemisch soweit zurück gedrängt werden, dass der Sauerstoff über den Luftschlauch in die Gasquelle gelangt, wo er durch seine hohen chemischen Reaktionseigenschaften in dem Bereich des Elektromotors der Gasquelle Brandschäden verursachen kann, bzw. eine teurere, feuerfeste Motorauslegung erforderlich macht.

Ein in dem Totraum der Atemmaske positionierter chemischer Sauerstoffsensor wird verwendet, um mit dem gemessenen Wert eine gewünschte Sauerstoffkonzentration einzustellen. Wegen der Trägheit des zumeist chemischen Sauerstoffsensors und der Länge des Luft zuführenden Schlauchs, die gewöhnlich etwa 2 m beträgt, kann dieses System jedoch nur im statischen Betrieb, d.h. ohne einen angeschlossenen Patienten, eine genaue Sauerstoffkonzentration gewährleisten. Die durch den Patienten ausgeatmete Luft verursacht Rückströme, die bis in den Luft zuführenden Schlauch wirken und das Atemgasgemisch mit dem CO2-Gas anreichern.

Abgesehen davon, verursachen die durch die Atemperiodizität bewirkten, vor- und rückwärts gerichteten Gasgemischbewegungen in dem Totraum der Atemmaske, dass dort der Sauerstoffgehalt stark in Abhängigkeit von Atemphasen variiert. Zur Kontrolle der Sauerstoffkonzentration werden Sauerstoff-Sensoren eingesetzt, die bevorzugt unmittelbar im Bereich des Totraums der Atemmaske positioniert werden. Diese chemischen Sauerstoff-Sensoren verfügen jedoch über eine große Zeitkonstante, die eine Trägheit in die Messergebnisse einbringt, wodurch keine Unterscheidung der Sauerstoffkonzentration zu verschiedenen Atemphasen möglich ist.

Die bekannten Lösungen für diese Probleme sind zum einen eine Zufuhr eines übermäßig mit Sauerstoff angereicherten Atemgasgemisches und zum anderen eine Regelung des Sauerstoffgehalts in Abhängigkeit von einem im Blut des Patienten gemessenen Sauerstoffgehalt. Im ersten Fall kann es neben den unwirtschaftlichen Aspekten zu einer Überversorgung mit Sauerstoff, einer sogenannten Überoxidierung, des Patienten führen und im zweiten Fall ist ein komplexeres System mit einer Erfassung des Sauerstoffgehalts im Blut des Patienten erforderlich. Außerdem besteht bei letzterem Verfahren eine noch längere Zeitkonstante zwischen einer Änderung der Sauerstoffkonzentration in dem Atemgasgemisch und einer durch sie bewirkten Änderung des Sauerstoffgehalts im Blut des Patienten, was zumindest in einer Einstellphase bei besonders schwer betroffenen Patienten unerwünscht sein kann, da hierbei der Patient als ein Messinstrument zum experimentellen Auffinden einer optimalen Einstellung für eine gewisse Zeit benutzt wird.

In der medizinischen Fachwelt hat sich ein Fachbegriff "Fi02" für den Sauerstoffgehalt in der Atemluft verbreitet. Die englische Aufschlüsselung für FiO2 ist "Fraction of inspired O2", was wörtlich "Anteil des eingeatmeten Sauerstoffs" bedeutet. Die Bemühungen aller technischen Lösungen und der Medizin lauten möglichst dahingehend, das FiO2 genauer vorgeben zu können, wobei hier eine mehr oder weniger große Differenz zwischen einem theoretisch vorgegebenen, einem gemessenen und dem tatsächlichen FiO2 besteht.

Die zum technologischen Hintergrund zu zählenden, berücksichtigten Veröffentlichungen seien hierbei ohne Einzelbeschreibung angeführt: WO 002005051280 A3, WO 000000045702 A1, US 000006761165 B2, US 000040060560 A1, CA 000001039144 A.

Ein weiteres Problem besteht darin, dass in der Beatmung überwiegend hochkonzentrierter Sauerstoff als Beatmungsgas verwendet wird. Insbesondere in der Notfallbeatmung, bei der das Beatmungsgerät mindestens einen Teil seiner Betriebsenergie aus der angeschlossenen Gasdruckflasche bezieht, geht eine große Verschwendung und ein erhöhter Kostenfaktor mit diesem Sachverhalt einher.

Um die Sauerstoffverschwendung einzudämmen, stellen einige Geräte bereits Beatmungsgas über einen einstellbaren Mix aus Sauerstoff und Umgebungsluft zur Verfügung. Im Allgemeinen ist eine adäquate Versorgung eines beatmungsbedürftigen Patienten mit 50% Sauerstoffanteil in dem Beatmungsgas für die meisten Patienten völlig ausreichend.

Technisch wird dies meist durch eine Venturidüse realisiert, welche den Venturi-Effekt zur Beimischung von Umgebungsluft dem Treibgas Sauerstoff ausnutzt. Der Sauerstoffstrom wird dabei von einer Gasquelle an die Venturidüse übergeben. Der aus der Venturidüse ausströmende Sauerstoff erzeugt eine Sogwirkung, die es ermöglicht, die Luft aus einer Umgebung anzusaugen. Eine einfache Einstellbarkeit der Sauerstoffdosierung wird dadurch unterstützt, dass die Venturidüse durch einen verstellbaren Ventilkegel reguliert wird. Diese und weitere Beispiele für eine oben genannte Venturidüse sind in der Schrift DE 10 2004 030 747 A1 bereits beschrieben.

Im Bereich der Sauerstofflangzeittherapie wird eine Einsparung von Sauerstoff durch so genannte Sauerstoffsparsysteme erreicht. Diese sind meist an kleinere Sauerstoffdruckflaschen oder Flüssigsauerstoffbehälter kleiner 2 Liter angeschlossen. Der Patient ist über eine Nasenbrille oder Atemmaske mit dem Sparsystem verbunden. Bei der Einatmung pflanzt sich der dabei produzierte Unterdruck bis zum Gerät fort, wird gemessen und zu Beginn der Einatmung ein einstellbarer Bolus abgeliefert.

Durch die im Einatemzyklus erfolgende frühzeitige Abgabe des Sauerstoffes gelangt dieser in die für den Sauerstoff und Kohlendioxidaustausch wirksamen Lungenareale mit Lungenfunktionsgewebe, die sogenannten Alveolen. Die restlichen Atemwege dienen lediglich dem Transport der Atemluft und sind nicht mit Lungenfunktionsgewebe ausgestattet. Bei diesen Systemen erhält man Sparraten von bis zu 5:1 gegenüber einem Dauerflowbetrieb.

Ein weiterer Stand der Technik wird in der EP 0 916 358 A2, der EP 0 659 445 A1, der WO 98/31382 A1, der US 4637384 A sowie der EP 1 334 741 A1 und der DE 196 26 924 C2 beschrieben.

Die vorliegende Erfindung stellt sich die Aufgabe, die Zubereitung eines Atemgasgemischs derart zu gestalten, dass der Sauerstoffgehalt des effektiv für die Einatmung bereitgestellten Atemgasgemisches, d.h Fi02, dynamisch genauer bestimmbar ist, ohne hierfür eine Messung des Sauerstoffgehalts im Blut des Patienten oder in dem Totraum der Atemmaske durchführen zu müssen. Zugleich soll auch das Problem der Rückführung des Sauerstoffs in die Gasquelle gelöst werden.

Diese Aufgabe wird hinsichtlich der bereitzustellenden Vorrichtung dadurch gelöst, dass die Vorrichtung über mindestens eine Erfassung des Volumenstroms mindestens eines der zu vermischenden Gase oder Gasgemische verfügt, und die Steuerung den mindestens einen erfassten Volumenstromwert des mindestens einen zu vermischenden Gases oder Gasgemischs verarbeitet, und den Volumenstrom mindestens eines der zu vermischenden Gase oder Gasgemische steuert.

Hinsichtlich des erfindungsgemäßen Verfahrens wird die Aufgabe dadurch gelöst, dass entlang der Transportleitung an zumindest zwei Punkten die Verteilung der Konzentration mindestens eines Anteils eines Gasgemisches ermittelt wird. Es wird erfindungsgemäß auf die Verwendung eines Sauerstoffsensors zum Bestimmen des Sauerstoffgehalts ganz verzichtet. Die für die erfindungsgemäße Steuerung der Vermischung mindestens zweier Gase, bzw. Gasgemische erforderlichen Messwerte werden erfindungsgemäß aus mindestens einer Erfassung des Volumenstroms mindestens eines der zu vermischenden Gase, bzw. Gasgemische gewonnen. Die hierfür erforderlichen Durchflussmessgeräte weisen sehr gute dynamische Messeigenschaften bei guter Genauigkeit auf, wodurch eine sehr zeitnahe Vermessung eines oder mehrerer Volumenströme ermöglicht wird.

Erfindungsgemäß werden nicht ausschließlich Volumenströme, sondern auch optional Massenströme bestimmt und gesteuert. Die Verwendung der Begriffe Volumenstrom / Massenstrom und Volumendurchfluss oder Massenfluss erfolgt im Wesentlichen synonym.

Ein weiteres wichtiges erfindungsgemäßes Merkmal ist der in den Luft zuführenden Schlauch verlegte Einleitungspunkt des zweiten zu vermischenden Gases, des Sauerstoffs, der zwischen den beiden Schlauchenden liegt. Die optimale Position dieses Einleitungspunktes wurde erfindungsgemäß in einem Modell experimentell ermittelt und das Volumen in dem Schlauch in diskrete Volumensegmente in diesem Modell rechnerisch aufgeteilt, die orts- und zeitabhängig jeweils eine berechenbare Sauerstoffkonzentration aufweisen.

Erfindungsgemäß wird mit Hilfe von mindestens einem Durchflussmessgerät der Volumendurchfluss mindestens eines der zu vermischenden Gase erfasst. Der Volumendurchfluss des zweiten Gases oder Gasgemischs kann ebenso in einer bevorzugten Ausführung mit einem Durchflussmessgerät erfasst werden, oder alternativ, in einer anderen Ausführung, als ein konstanter Volumendurchfluss vorgegeben sein, der evtl. in mehreren konstanten Stufen verstellbar ist, um hierdurch eine Anpassung des Atemgemischvolumens durchführen zu können.

Der mindestens eine erfasste Messwert des Volumendurchflusses wird einer Steuerung zugeführt, in der aus dem oder den Messwerten das Volumen des einen oder beider zu vermischenden Gase berechnet wird. Die Information über das Volumen des zweiten beteiligten Gases/Gasgemischs liegt der Steuerung dann entweder als eine Konstante oder als ein erfasster Messwert vor.

Da die Erfassung mittels der Durchflussmessgeräte dynamisch sehr gut dem zeitlichen Ablauf von Inspiration- und Expirations-Phasen (Ein- und Ausatmen) folgen kann, lassen sich aus den gewonnenen zeitabhängigen Signalen u.a. auch Schlüsse auf die Atemfrequenz und dem Atemluftbedarf des Patienten ziehen.

Beim Ausatmen verursacht die von dem Patienten unter einem gewissen Druck entgegen dem in den Totraum der Atemmaske zugeführten Atemgasgemisch ausgeblasene Atemluft, dass sich dort ein Stau aus den beiden Gasgemischströmen ergibt. In dieser Phase verlangsamt sich der Zustrom des Atemgasgemischs aus dem Schlauch, was die Durchflussmessgeräte in einem verringerten Volumendurchfluss feststellen. Die Steuerung erkennt anhand des oder der verringerten Volumendurchflüsse und der berechneten Volumina die Atemfrequenz und den Atemluftbedarf des Patienten, und regelt, in einer bevorzugten Ausführung, den Zufluss eines oder beider zu vermischender Gase entsprechend den gemessenen und ermittelten Größen.

Der Algorithmus zur Bestimmung der Regelgrößen für die Regelventile benutzt sowohl eine Zeit-, wie Ortsdiskretisierung der Volumenabschnitte in dem gesamten das Gasgemisch zuführenden System, hauptsächlich dem Schlauch und dem Totraum der Atemmaske, und geht hierfür von den gemessenen Volumendurchflüssen beider beteiligten Gase, bzw. Gasmischungen aus.

Die gemessenen Volumendurchflüsse sind durch die Ausatmungsphasen des an das Beatmungssystem angeschlossenen Patienten zeitabhängig, wodurch deren Größen nach einem Integrationsrechenschritt über die Zeit das jeweilige Volumen für den betrachteten Zeitraum liefern. Untergliedert man die Integrationszeitabschnitte in kleine diskrete Abschnitte, dann erhält man die zugehörigen pro Zeitperiode ins System eingeleiteten Volumina der beiden beteiligten Gase. Durch Referenzmessungen der Sauerstoffkonzentration entlang eines in dem Zuführungsschlauch gebildeten Modells bei verschiedenen simulierten Volumendurchflüssen erhält man eine experimentelle Entsprechung der Konzentrationsentstehung und Verteilung in Abhängigkeit von den Volumendurchflüssen der einzelnen Gase. Hinzu kommt, dass eine dritte Gasmischung, nämlich die ausgeatmete Luft, periodisch entgegen dem Haupfluss der Atemgasmischung in den Modellraum einströmt und durch den erhöhten CO2-Gehalt die Konzentration des Sauerstoffs insbesondere in dem Totraum der Atemmaske periodisch herabsetzt.

Eine weitere wichtige Rolle kommt dem Entlüftungssystem zu, durch welches die ausgeatmete Luft in die Umgebung entweichen kann. Es sind Ventile, definierte Lecköffnungen oder Diffusionsfilter hierzu im Einsatz. Der Algorithmus errechnet die entweichende Luftmenge aus dem Gesamtfluss der Atemgasmischung, da diese zusammen mit der ausgeatmeten Luft sämtlich entweichen muss.

Durch Referenzmessungen im selben Model können mit den CO2-Sensoren ebenso die Konzentration und ihre zeitliche, wie örtliche Verteilung im Atemgas führenden System für verschiedene Betriebsregime ermittelt werden und in einer mathematischen Abbildung eine Berücksichtigung in dem Algorithmus finden.

In einer bevorzugten Ausbildung kann der Volumendurchfluss auch des Sauerstoffs geregelt werden, so dass während der Expirationsphase kein Sauerstoff und in der Inspirationsphase dafür gezielt eine genau benötigte Menge von Sauerstoff beigemischt wird.

Als Resultat erhält man ein Vermischungssystem für zwei Gase oder Gasmischungen, das wesentlich genauer ohne Verwendung einer zu trägen chemisch-sensorischen Bestimmung der Sauerstoffkonzentration auskommt, wodurch bei entsprechend sorgfältiger Auslegung des Systems eine verbesserte Genauigkeit der Versorgung eines Patienten mit der für ihn richtigen Sauerstoffmischung ermöglicht wird.

Ferner lassen sich die gleichen Prinzipien und Algorithmen nach der vorliegenden Erfindung auch auf andere technische Gebiete und Systeme übertragen, so auch auf flüssige Stoffe, für mehr als zwei Stoffe und für verschiedenste Anwendungen, bei denen chemisch arbeitende Sensoren zu träge für eine dynamisch veränderliche Mischungsgröße sind, so dass eine Anwendung möglich ist. Ebenfalls kann eine Anwendung in Fällen erfolgen, in denen das System über definierte geometrische und auch durchaus recht komplexe Gestalt gebildet ist, da die Strömungsverhältnisse im wesentlichen erfindungsgemäß mathematisch abgebildet werden können.

Mit der vorliegenden Erfindung lassen sich unter solch komplexen und ähnlichen Bedingungen, wie einem Beatmungssystem, welches über eine entgegen strömende Exspirationsluft verfügt, wesentlich genauere Fi02-Werte gemäß den Vorgaben einhalten, als es konventionelle Systeme zu erreichen vermögen.

Hinsichtlich der Einsparung von Sauerstoff besteht eine Aufgabe darin, ein Verfahren und eine Vorrichtung der einleitend genannten Art derart zu verbessern, dass die Verbrauchsmenge an Sauerstoff reduziert wird.

Hinsichtlich des nachfolgenden nicht erfindungsgemäßen Verfahrens wird diese Aufgabe dadurch gelöst, dass für einen Zeitbereich einer frühen Inspirationsphase eine hohe Sauerstoffkonzentration im Vergleich zu einer restlichen Inspirationszeit am Ende einer Beatmungsphase bereitgestellt wird.

Hinsichtlich der Vorrichtung wird diese Aufgabe dadurch gelöst, dass die Steuer- und Auswerteeinheit ein Steuerprogramm zur Vorgabe einer Sauerstoffdosierung derart aufweist, dass für einen Zeitbereich einer frühen Inspirationsphase eine hohe Sauerstoffkonzentration im Vergleich zu einer restlichen Inspirationszeit am Ende einer Beatmungsphase vorgegeben ist.

Vorteil des Verfahrens ist, dass lediglich der Anteil des Atemgases mit hoher Sauerstoffkonzentration bereitgestellt wird, welcher auch physiologisch zur Oxygenierung des Blutes sinnvoll genutzt werden kann. Das weiterhin zur Ventilation nötige Atemgas wird optimiert in der Hinsicht, dass der eingesetzte Sauerstoff minimiert wird. Daraus erwächst eine deutlich verlängerte Einsatzdauer des verfügbaren Drucksauerstoffs.

Mit optimierten Injektoren nach dem Venturiprinzip zur Ansaugung von Umgebungsluft ist eine Verlängerung der Sauerstoffverfügbarkeit um den Faktor 2 bis 4 möglich.

Durch eine Dosierung von höchstkonzentriertem Sauerstoff zu Beginn einer Inspiration wird eine bestmögliche Versorgung der Alveolen mit Sauerstoff erzielt, auch wenn im nachfolgenden Verlauf der Inspiration die Sauerstoffkonzentration verringert wird. Diese Verringerung kann durch Beimischung von z.B. Umgebungsluft oder Luft aus Haussystemen, wie sie in Krankenhäusern vorzufinden sind, durch ein Venturiprinzip o.ä. erfolgen. Dieses Gas mit verringerter Sauerstoffkonzentration dient in erster Linie dazu, um den Bolus hoher 02-Konzentration bis zu den Alveolen zu transportieren, wobei es selbst nur die Atemwege und nicht die Areale mit Lungenfunktionsgewebe erreicht.

In einer Ausführungsform kann die zeitlich begrenzte Abgabe der hohen Sauerstoffkonzentration zu Beginn der Inspiration unabhängig von den Beatmungsparametern erfolgen. Der Zeitbereich der erhöhten Sauerstoffabgabe kann fest vorgeben werden.

In einer weiteren Ausführungsform kann die begrenzte Abgabe der hohen Sauerstoffkonzentration in Abhängigkeit von Beatmungsparametern erfolgen. Dabei kann beispielsweise eine beliebige Kombination aus Inspirationszeit, Inspirationsflow und Inspirationsdruck sowie generell Druck-, Flow- und Volumen-Kriterien, beispielsweise in Abhängigkeit von der Inspirationszeit Ti, in unterschiedlichen Gewichtungen zur Festlegung des Zeitbereiches für die Abgabe der hohen Sauerstoffkonzentration herangezogen werden.

Ebenso können weitere physiologische Parameter wie z.B. Hämoglobinwert, et CO2, spO2 oder weitere direkte oder indirekte Indikatoren für eine Oxygenierung des Blutes genutzt werden. Ferner können gerätetechnische Parameter wie Schlauchvolumen, Volumen der gesamten Beatmungsstrecke, Volumen Geräteblock, pneumatische Widerstände der Beatmungsgerätkomponenten sowie weitere zur Berechnung des richtigen Applikationszeitpunktes des Sauerstoffes notwendige technische Parameter in das Verfahren einfließen.

Der Zeitpunkt der Auslösung des Bolus ist durch das Volumen zwischen dem Ort der Sauerstoffeinspeisung und dem Ort des Gemischausganges mit einer Vorlaufzeit t_x versetzt vorher auszulösen. Dazu sind unter anderem Flow und Beatmungsstreckenvolumen auszuwerten.

In einer weiteren Ausführungsform werden die physiologischen, beatmungsrelevanten Parameter mit verschiedenen Sensoren erfasst und einer Trendanalyse unterzogen. Diese versucht, die nächsten Atembewegungen des Patienten vorherzusagen, um die Vorlaufzeit t_x optimal zu berechnen.

Ausführungen der vorliegenden Erfindung werden nun mit Bezug auf die zugehörigen Zeichnungen lediglich beispielhaft beschrieben, wobei funktionell gleiche Teile mit entsprechenden gleichen Bezugskennziffern bezeichnet sind.
- Fig. 1: zeigt eine prinzipielle Anordnung eines Volumenmodels einer bevorzugter Ausführung vorliegender Erfindung,
- Fig. 2: zeigt ein Flussdiagramm des Algorithmus zur Berechnung der Austrittskonzentration und des FiO2 der bevorzugten Ausführung vorliegender Erfindung,
- Fig. 3: zeigt einen Algorithmus der FiO2-Regelung,
- Fig. 4: zeigt ein Blockschaltbild der FiO2-Regelung,
- Fig. 5: zeigt eine graphische Darstellung des Volumendurchflussverlaufs,
- Fig. 6: zeigt eine schematische Anordnung eines erfindungsgemäßen Beatmungssystems,
- Fig. 7: zeigt einen Längsschnitt durch einen Venturiinjektor mit angeschlossenen Funktionskomponenten,
- Fig. 8: zeigt eine Darstellung einer Beatmungsphase mit erfindungsgemäßer Sauerstoffdosierung,
- Fig. 9: zeigt eine gegenüber Fig. 8 abgewandelten Darstellung unter Berücksichtigung einer Vorlaufzeit und
- Fig. 10: zeigt eine Darstellung mit konstanter Sauerstoffdosierung während der gesamten Inspirationsphase.

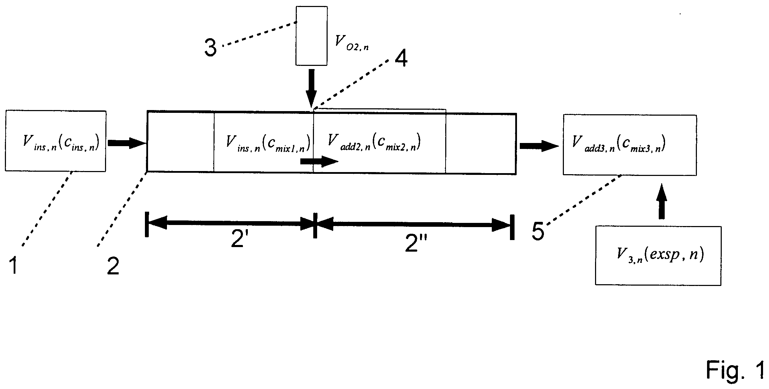

In Figur 1 ist eine prinzipielle Anordnung eines Volumenmodels einer bevorzugter Ausführung vorliegender Erfindung zu sehen. Ganz allgemein erklärt, wird aus einer Quelle 1 ein nicht fester Stoff erster Art, ein flüssiger oder gasförmiger, mit einem Volumen Vins,n(cins,n) in einen relativ zu seinem Durchmesser länglich ausgebildeten Mischraum 2 mit einem bestimmten Volumendurchfluss unter Druck eingegeben. In einem Einleitungspunkt 4 wird in den Mischraum 2 ein nicht fester Stoff zweiter Art, ein flüssiger oder gasförmiger, mit einem Volumen V02,n unter Druck eingeleitet. Aus dem Mischraum 2 tritt ein Gemisch aus den zwei nicht festen Stoffen mit einem Volumen Vadd3,n(cmix3, n) heraus.

Der nicht feste Stoff erster Art mit dem Volumen Vins,n(cins,n) weist eine Konzentration c1 des nicht festen Stoffes zweiter Art auf. In der bevorzugten Ausführung ist der Stoff zweiter Art der reine Sauerstoff (bzw. ein hochkonzentriertes Sauerstoff-Gemisch) und der Stoff erster Art ist ein Gasgemisch, das im wesentlichen der atmosphärischen Luft entspricht. Vorzugsweise ist der Einleitungspunkt als ein Rohr ausgeführt, der in Flussrichtung in dem Mischraum 2 angeordnet ist. Die Zahl n in Klammern bei dem Volumen Vadd2,n(cmix2,n) steht für ein gedachtes diskretes Teilvolumen, dessen Konzentration des zweiten in ihm enthaltenen nicht festen Stoffs, hierbei des Sauerstoffs, eine orts- und zeitabhängige Variable cmix ist.

Am Ausgang aus dem Mischraum 2 tritt das Gemisch aus den zwei vermischten Stoffen erster und zweiter Art mit einem gesamten Volumen Vadd3,n(cmix3,n) heraus, welches eine Konzentration des zweiten nichtfesten Stoffs von cmix3,n aufweist, die ebenso in diskreten Volumenmengen-Schritten n sich in einem Fertiggemisch-Raum 5 orts- und zeitabhängig verändert. Ein nicht festes Stoffgemisch dritter Art mit dem Volumen V3,n(cexp,n) ist in den Fertiggemisch-Raum 5 eingeleitet. In der bevorzugten Ausführung ist es die ausgeatmete Luft einer Person, wobei die Luft periodisch zeit- und ortsabhängig ihre Richtung, Sauerstoff-, CO2-Gehalt usw. verändert.

Nicht eingezeichnet sind die Volumendurchfluss oder Massen stromsensoren, die die beiden, bzw. zumindest einen von beiden, Volumenströme der beiden zu vermischenden Stoffe messen, sowie Regelstelleinheiten, wie elektromechanisch gesteuerte Ventile, mittels derer eine Steuerung mindestens einen der Volumenströme in Abhängigkeit von gemessenen und vorliegenden Volumenströmen bzw. Massenströmen steuert.

Des Weiteren sind zwei weitere Abschnitte des Mischraums 2 aufgezeigt: Vorraum 2' und Nachraum 2" bezogen auf den Einleitungspunkt 4

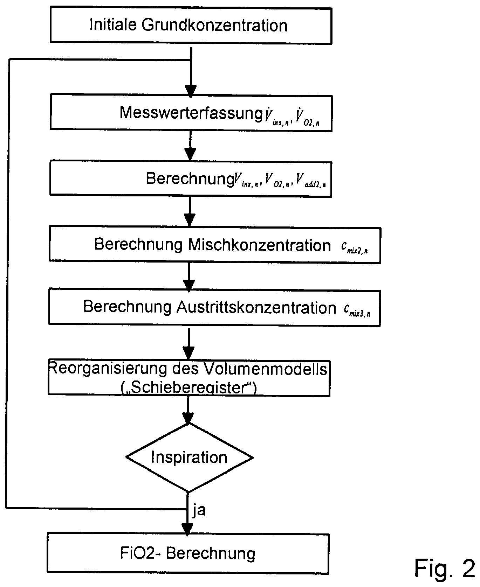

In Figur 2 ist ein Flussdiagramm des Algorithmus zur Berechnung der Austrittskonzentration und des FiO2 der bevorzugten Ausführung nach vorliegender Erfindung zu sehen.

Dem Prozess liegen zunächst die Werte für eine jeweilige Grundkonzentration in den beiden Abschnitten des Mischraums 2, zugrunde einem Wert im Vorraum 2' und einem Wert im Nachraum 2" bezogen auf den Einleitungspunkt 4.

Im ersten Prozessschritt werden die Messwerte der Volumenströme *V̇*_{*insp*,*n*}, *V̇*_{*O*2,*n*} mittels Volumendurchflusssensoren erfasst, bzw. mindestens ein Wert als Messwert erfasst, wenn der andere Wert als ein konstanter Durchfluss vorliegt. Da die Volumenströme erste zeitabhängige Ableitungen des zugehörigen Volumens sind, wird in einem nächsten Schritt aus den gemessenen Volumenströmen jeweils das zugehörige Volumen *V*_{*insp*,*n*}, *V*_{*O*2,*n*}, *V*_{*add*2,*n*} in einer Steuerung berechnet. Mit den dann vorliegenden Werten wird in der Steuerung die Mischkonzentration *c*_{*mix*2,*n*} berechnet. Ferner wird in der Steuerung nach einem geeigneten Algorithmus ortsabhängig die Konzentration des Sauerstoffs für diskrete Volumen berechnet. Abhängig von den Inspirations- und Exspirationsphasen wird daraus eine Volumenverschiebung berechnet und als Resultat eine Austrittskonzentration *c*_{*mix*3,*n*} für die fertige Gasmischung am Austrittsende des Mischraums berechnet, die zeitabhängig die Verschiebung der ortsdiskreten Volumenabschnitte verschiedener Konzentrationen berechnet.

Die austretende Gasmischung wird, wenn gerade zeitablauftechnisch mit der Beatmung der Person passend, in einer Inspirationsphase eingeatmet. Falls diese Bedingung nicht vorliegt, wird in dem Ablauf an den Anfang verzweigt.

Für die Berechnung des FIO2, d.h. des Sauerstoffgehalts in der Atemgasmischung, wird in einer bevorzugten Ausführung ein am Schluss des Diagramms gezeigter Algorithmus verwendet. Weitere genauere Algorithmen sind denkbar, sowohl aus theoretischen, wie experimentellen Ansätzen.

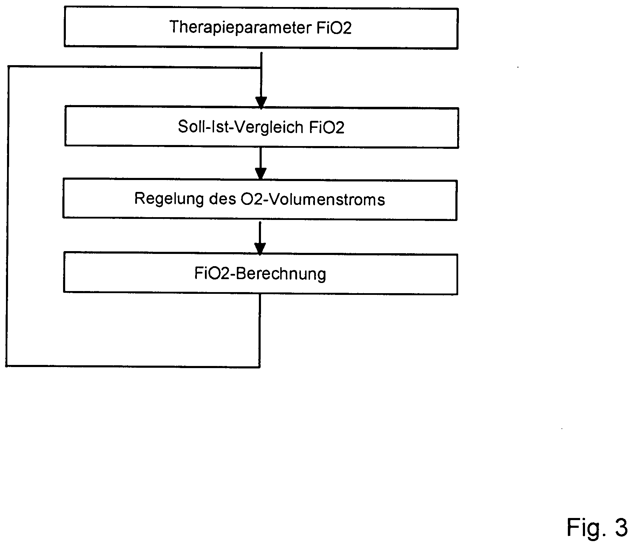

In Figur 3 ist ein Algorithmus der Steuerung des Sauerstoffgehalts, des FIO2, der Atemluft zu sehen.

Als Eingangsgrößen liegen die Therapieparameter für FiO2 vor, die beispielsweise ein Arzt für einen bestimmten Patienten vorschreibt. Diese werden mittels einer Eingabeeinheit in das System eingegeben.

Im nächsten Verfahrensschritt wird ein Vergleich zwischen dem vorgegebenen FiO2-Sollwert und dem ermittelten FiO2-Istwert durchgeführt und aus diesem Vergleich werden ein oder zwei Werte für einen anschließend folgenden Verfahrensschritt bereit gestellt, in dem eine Regelung des Sauerstoff-Volumenstroms durchgeführt wird. Für die Ausführung dieser Regelung des Volumenstroms sind in einer bevorzugten Ausführung elektromechanisch angetriebene Ventile oder Stelleinheiten vorgesehen, die jeweils einen der beteiligten nichtfesten Stoffe, hierbei im Beispiel Gase und Gasmischungen, vorgesehen.

In einer vereinfachten bevorzugten Ausführung wird nur einer der beiden zu vermischenden Stoffe mit einer Regelung, d.h. einem elektromechanischen Ventil oder einer Stelleinheit, ausgestattet, während für den Volumenstrom des zweiten beteiligten Stoffes eine konstante Größe vorgegeben wird, die durch eine kalibrierte Durchlassöffnung strömungshydraulisch vorgegeben ist.

Im nächsten Verfahrensschritt steht die zubereitete Atemgasmischung der Beatmung des Patienten zur Verfügung.

Der nächste folgende Verfahrensschritt beinhaltet die unter Figur 2 beschriebene Berechnung des FiO2-Wertes aus den gemessenen Volumendurchflusswerten, woraus die FiO2-Istwerte für den geschlossenen Regelkreis ermittelt werden und wieder an seinen Anfang dem Verfahrensschritt zugeführt werden, in dem ein Sollwert-Istwert-Vergleich stattfindet.

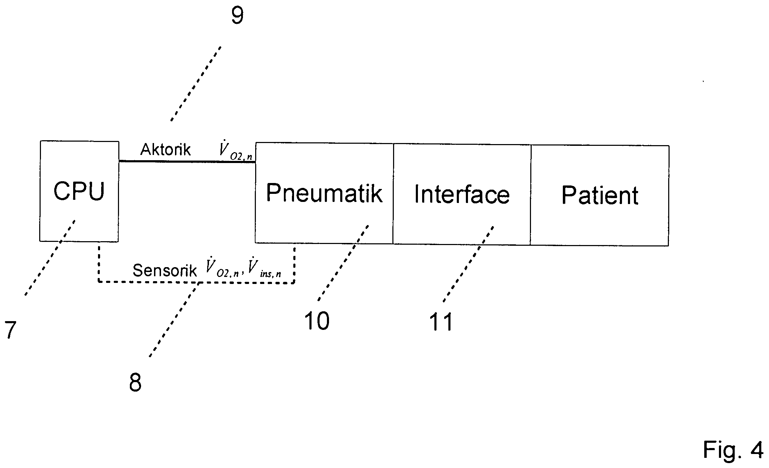

In Figur 4 ist ein Blockschaltbild der FIO2-Regelung nach einer bevorzugten Ausführung dargestellt. Es schließt als ein Teilelement einen Patienten 12 mit ein, und umfasst eine Steuerung 7 mit einer in ihr enthaltenen Recheneinheit (CPU), eine pneumatische Einheit 10, eine Benutzerschnittstelle 11, die in der bevorzugten Ausführung der Erfindung einer Atemmaske entspricht, und einer zwischen der Steuerung 7 und der pneumatischen Einheit geschalteten Sensorikeinheit 8, sowie einer Aktorikeinheit 9.

Die pneumatische Einheit 10 umfasst alle die Pneumatik betreffenden Teile, wie den Mischraum, der in einer bevorzugten Ausführung als ein Schlauch gestaltet ist und einen zweiten Zuführungsschlauch, über den der Sauerstoff in den Mischraum eingespeist wird, sowie kalibrierte Öffnungen, die strömungshydraulisch für einen vorgegebenen normalen oder maximalen Volumendurchfluss sorgen.

Die Steuerung 7 umfasst eine Recheneinheit, die in der bevorzugten Ausführung mit den üblichen Mitteln zum Laden, Vorhalten und Zwischenspeichern von Daten, sowie einer datentechnischen Peripherie-Schnittstelle ergänzt ist. Sie empfängt über diese Peripherie-Schnittstelle von den Volumendurchflusssensoren die gemessenen Signale und verarbeitet sie nach einem programmtechnisch in der Recheneinheit ablaufenden Algorithmus. Als Ergebnis gibt die Steuerung 7 über einen Ausgabeteil der Peripherie-Schnittstelle den rechnerisch ermittelten Regel-Stellgrößen entsprechende elektrische Signale für eine oder mehrere Stelleinheiten aus, die den Volumendurchfluss eines jeweiligen zu vermischenden Stoffes regeln.

Die Benutzerschnittstelle 11 ist in der bevorzugten Ausführung der vorliegenden Erfindung eine Atemmaske, die als Bindeglied zwischen dem ein Atemgasgemisch zubereitenden System und dem Patienten dient.

Die Sensorikeinheit 8 umfasst den oder die Volumendurchfluss-Sensoren, sowie dazu gehörende Leitungen und evtl. benötigte Signalumsetzer. Als Volumendurchfluss-Sensoren eignen sich vorzugsweise Hitzedraht- oder Differenzdrucksensoren, die über gute dynamische Eigenschaften verfügen. Die Aktorikeinheit 9 umfasst ein oder mehrere elektromechanisch angetriebene Regelventile, bzw. Stelleinheiten, die den Volumendurchfluss variieren können, sowie dazu gehörende Leitungen und evtl. benötigte Signalumsetzer.

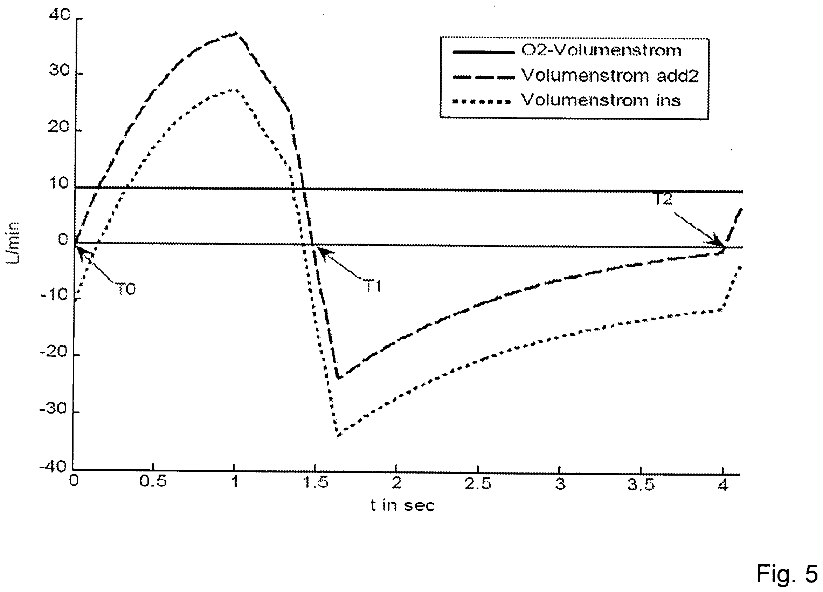

In Figur 5 ist eine graphische Darstellung des Volumendurchflussverlaufs zu sehen. In dieser bevorzugten Ausführung ist der Volumendurchfluss des Sauerstoffs konstant vorgegeben, hierbei dargestellt mit der durchgezogenen Linie. Die gepunktete Linie stellt den Volumendurchfluss der mit dem Sauerstoff zu vermischenden Luft dar, und die gestrichelte Linie zeigt den Verlauf des gesamten summierten Volumendurchflusses und/oder O2-Regelung der zubereiteten Atemgasmischung. Es ist ersichtlich, dass zwischen der Startzeit und der Zeit T1 eine Inspiration, d.h. ein Einatmen, stattfindet, und zwischen der Zeit T1 und T2 die Exspiration oder ein Ausatmen stattfindet. Die Steuerung regelt hierbei den Volumendurchfluss in Abhängigkeit von der aus den gemessenen Durchflusswerten ermittelten Atemfrequenz hoch und runter.

In einer weiteren bevorzugten Ausführung ist auch der Durchfluss des Sauerstoffs abhängig von den Atemphasen gesteuert - dann erhalten alle drei Kurvenverläufe eine ähnliche Gestalt.

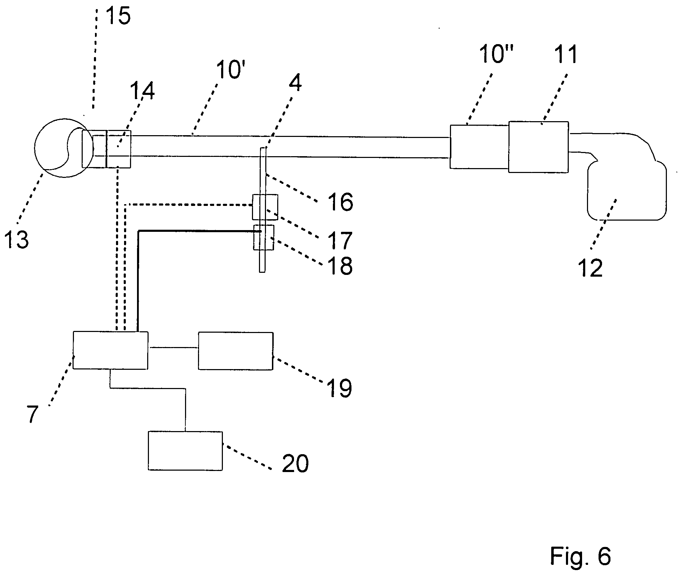

Figur 6 zeigt eine schematische Anordnung einer bevorzugten Ausführung des erfindungsgemäßen Beatmungssystems.

Das System besitzt eine Gasquelle 13, welche eines der zu vermischenden Stoffe, hierbei die Umgebungsluft, komprimiert und in den Schlauch 10' einleitet. Der zweite zu vermischende Stoff, der hierbei Sauerstoff ist, ist über eine eigene Zuleitung 16 in den Schlauch 10' an einem günstig positionierten Einleitpunkt 4, der sich zwischen den Enden des Schlauchs 10' befindet, eingeleitet. Der Sauerstoff wird entweder, wie zumeist üblich, aus einer Gasflasche über einen Druckminderer entnommen, oder von einem Erzeugergerät direkt geliefert (beide sind nicht dargestellt).

Die Zufuhr der Umgebungsluft wird mit dem vorzugsweise am Anfang des Schlauchs 10' positionierten Volumendurchflusssensor 14 erfasst und mit einem elektromechanisch angetriebenen Regelventil 15 gesteuert, dessen Durchflussöffnung sich verstellen lässt.

In dem Schlauch 10' zwischen seinen beiden Enden ist der Einleitpunkt 4 der Zuleitung 16 positioniert, über den der Sauerstoff eingeleitet wird. Die Zuleitung 16 für die Sauerstoffzufuhr weist in dieser bevorzugten Ausführung ebenso einen Volumendurchflusssensor 17 und ein elektromechanisch angetriebenes Regelventil 18 auf, die eine Steuerung des Volumendurchflusses des Sauerstoffs ermöglichen.

Die Signale der Volumendurchflusssensoren 14 und 17 werden der Steuerung 7 zugeführt, die sie auswertet und nach einem Algorithmus berechneten Stellwerte für die ebenso an sie angeschlossenen elektromechanisch angetriebenen Regelventile 15 und 18 ausgibt. Die Steuerung 7 gibt außerdem Messwerte und/oder berechnete Werte auf einer Anzeigevorrichtung 19 zu Kontrollzwecken aus. Für die manuelle Vorgabe von Sollwerten für die Sauerstoffkonzentration, des FiO2-Wertes, ist eine Eingabeeinheit 20 vorgesehen.

Am Ende des Schlauchs 10' befindet sich ein Ausatemsystem 10", das zumeist entweder als ein Diffusionsfilter oder eine Öffnung realisiert ist. Unmittelbar daran anschließend folgt eine Benutzerschnittstelle 11, die in der vorliegenden bevorzugten Ausführung als eine Atemmaske ausgeführt ist. Diese Benutzerschnittstelle ist an die Atemorgane eines Patienten 12 angeschlossen und sorgt für eine komfortable erträgliche Anbindung des Menschen an das Beatmungssystem.

Das Verfahren ist mittels eines in der Steuerung 7 ablaufenden Programms realisiert, welches anhand gemessener Volumendurchflusswerte für den einen oder beide zu vermischende Stoffe deren Volumina im zeitlichen und örtlichen Verlauf erfasst, daraus nach einem Algorithmus den Verlauf der FiO2-Konzentration im Schlauch 10', dem Ausatemsystem 10" und der Benutzerschnittstelle 11 berechnet und auf der Anzeigevorrichtung 19 anzeigt.

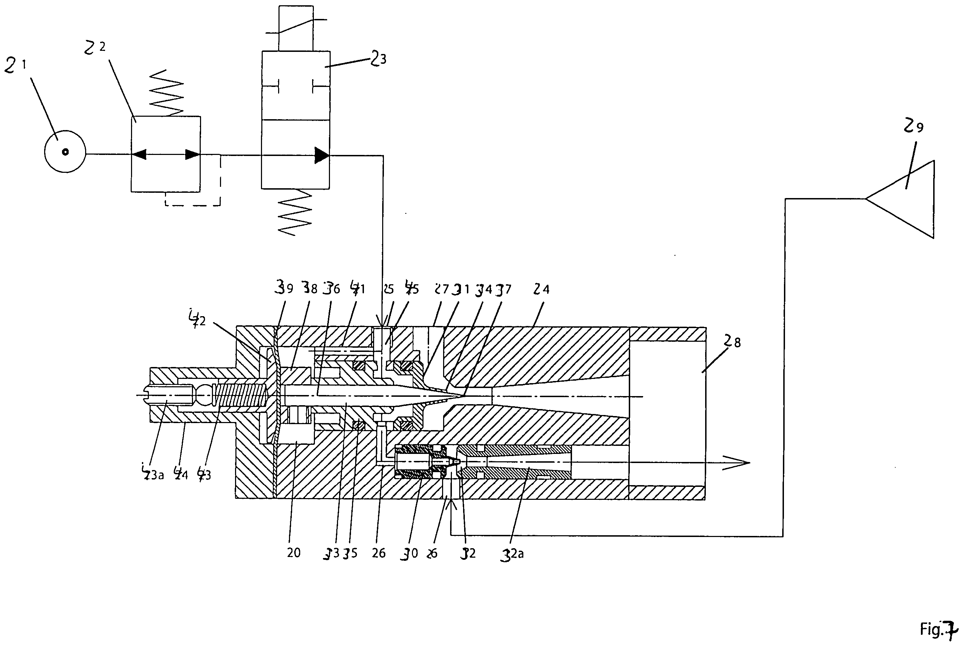

Fig. 7 zeigt eine Druckgasquelle (21), die über einen Druckregler (22) und ein Steuerventil (23) an eine Mischeinrichtung (24) angeschlossen ist. Die Mischeinrichtung (24) besitzt einen Druckgaseingang (25), mindestens einen Umgebungsluftanschluß (26, 27) sowie einen Ausgang (28).

Die Druckgasquelle (21) kann beispielsweise als eine Flüssiggasquelle ausgebildet sein, deren Druck vom Druckregler (22) auf einen Bereich von 2,7 bis 6 bar eingestellt wird. Das Steuerventil (23) kann als ein Proportionalventil ausgebildet sein. Den Umgebungsluftanschlüssen (26, 27) wird die Umgebungsluft vorteilhafterweise über einen Luftfilter (29) zugeführt.

Die Mischeinrichtung (24) gemäß Fig. 1 weist zwei Venturidüsen (30, 31) auf, wobei die Venturidüse (30) eine Grundstromstufe für einen unteren Lastbereich und die Venturidüse (31) eine Vollstromstufe für einen oberen Lastbereich bereitstellt. Insbesondere ist daran gedacht, im oberen Lastbereich die Venturidüsen (30, 31) relativ zueinander parallel zu betreiben.

Die Venturidüse (30) ist als ein Festinjektor ausgebildet, dem über den Druckgaseingang (25) das Druckgas als Treibgas zugeführt wird. Die Umgebungsluft wird über den Umgebungsluftanschluß (26) zur Venturidüse (30) geleitet. Aufgrund der Injektorwirkung der aus der Venturidüse (30) ausströmenden Gasströmung an Druckgas wird Umgebungsluft mitgerissen, im Bereich einer Mischkammer (32) mit dem Druckgas vermengt und anschließend in Richtung auf den Ausgang (28) geleitet. Dabei wird über einem Diffusor (32a) erneut Druck aufgebaut.

Die Venturidüse (31) ist als ein Nadelinjektor ausgebildet, wobei bei in dem in Fig. 1 dargestellten Betriebszustand eine Nadel (33) den Düsenausgang (34) verschließt. Die Nadel (33) ist von einem Nadelträger (35) gehaltert, in dem die Nadel (33) in Richtung einer Nadellängsachse (16) beweglich geführt ist. Eine Nadelspitze (37) ragt aus dem Düsenausgang (34) heraus. Ein der Nadelspitze (37) abgewandter Nadelsockel (38) ist auf einer Membran (39) befestigt, die in einer Membrankammer (40) angeordnet ist. Die Membrankammer (40) ist über einen Steuerkanal (41) mit dem Druckgaseingang (25) verbunden. Darüber hinaus ist die Membran (39) im Bereich ihrer der Membrankammer (40) abgewandten Oberfläche von einem Gegenelement (42) abgestützt, das über eine Feder (43) gegenüber einem Kammergehäuse (44) verspannt ist.

Die Kraft der Feder (43) ist derart dimensioniert und mit Hilfe einer Einstellschraube (43a) so voreingestellt, dass bei einem am Druckgaseingang (25) anliegenden unteren Druckbereich, der über den Steuerkanal (41) auf die Membrankammer (40) übertragen wird, die Kraft der Feder (43) ausreichend ist, um die Nadel (33) abdichtend in den Düsenausgang (34) zu drücken.

Zur Unterstützung einer kompakten Ausbildung der Mischeinrichtung (24) verläuft ein Verbindungskanal (45) ausgehend vom Druckgaseingang (25) am Nadelträger (15) vorbei bis zu einem Eingangskanal (46) der Venturidüse (30). Hierdurch kann eine gemeinsame Druckgasversorgung beider Venturidüsen (30, 31) erreicht werden.

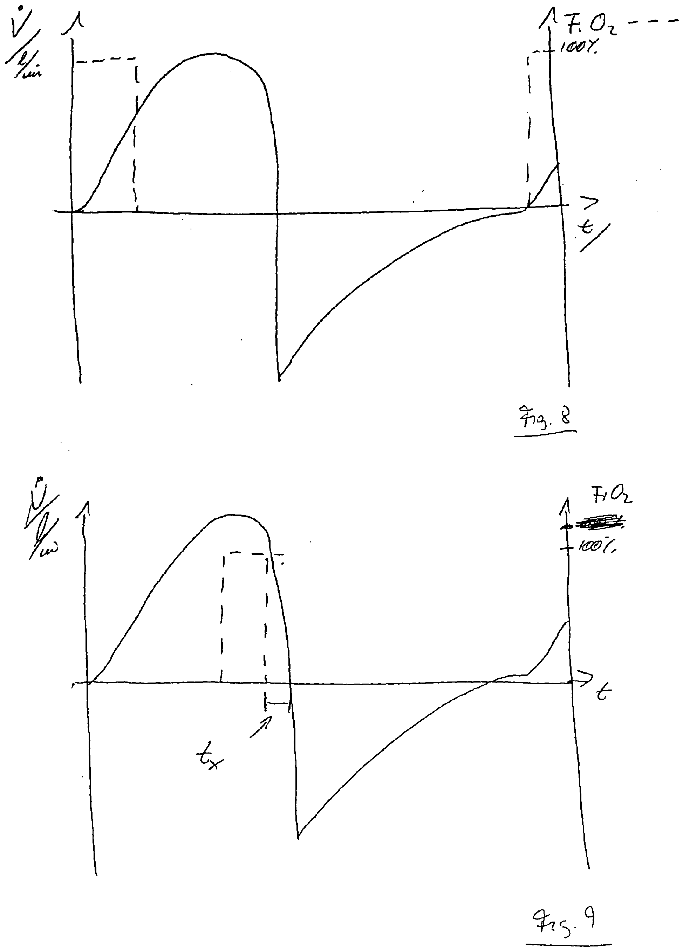

Fig. 8 zeigt einen Verlauf eines Beatmungszyklus mit einer Inspirationsphase und einer Exspirationsphase. Während eines Teiles der Dauer des Exspirationsphase wird Sauerstoff zudosiert. Die Zeitspanne der Zudosierung ist durch den gestrichelten Verlauf gekennzeichnet. Es ist zu erkennen, dass die Sauerstoffdosierung unmittelbar mit dem Beginn der Inspirationsphase startet und noch innerhalb der Inspirationsphase zu einem geeigneten Zeitpunkt beendet wird, an dem die Effektivität einer Sauerstoffaufnahme durch den Patienten zurückgeht.

Fig. 9 zeigt eine gegenüber der Darstellung in Fig. 8 abgewandelte Dosierung des Sauerstoffes. Die Dosierung beginnt hier unter Berücksichtigung einer Vorlaufzeit TX, die die Laufzeit der der Sauerstoffeinspeisung zugeordneten Volumeneinheiten durch das Beatmungssystem hindurch bis in den Bereich des Applikationsortes berücksichtigt.

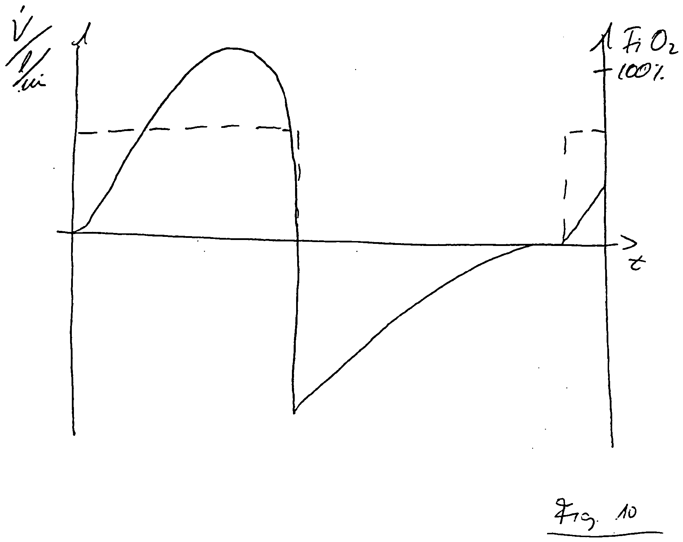

Fig. 10 zeigt eine Sauerstoffdosierung, die sich über den gesamten Zeitraum der Inspirationszeit erstreckt. Es ist vorgesehen, dass die Atemphasen, insbesondere das Ein- und Ausatmen, des Patienten erfasst werden und der Volumenstrom und/oder Massenstrom zumindest eines der zu vermischenden Gase oder Gasgemische in Abhängigkeit von den Atemphasen gesteuert wird. Es ist vorgesehen, dass die Atemphasen durch die Erfassung des Volumenstroms und/oder Massenstroms mindestens eines der zu vermischenden Gase oder Gasgemische und eine Auswertung der Messergebnisse durchgeführt wird. Es ist vorgesehen, dass aus den erfassten Messwerten und berechneten Größen und deren zeitlichem Verlauf der Atemluftbedarf des Patienten berechnet und die Zufuhr der zubereiteten Atemgasmischung abhängig davon gesteuert wird. Es ist vorgesehen, dass am Ende der Transportleitung die Konzentration mindestens eines Anteils eines Gasgemisches ermittelt wird. Es ist vorgesehen, dass ein Vergleich des vorgegebenen Sollwertes und des berechneten Istwertes der Mischkonzentration durchgeführt wird, und dass mit dem Vergleichsergebnis eine Regelung des Volumenstroms und/oder Massenstroms mindestens eines der beteiligten nicht festen Stoffe, vorzugsweise Gase oder Gemische, durchgeführt wird. Es ist vorgesehen, dass mindestens teilweise Sauerstoff von einer Sauerstoffquelle und Luft zu einem Gasgemisch zusammengeführt werden, wobei die Sauerstoffkonzentration (Zusammensetzung) des Gasgemisches zeitlich variiert werden kann, dadurch gekennzeichnet, dass für einen Zeitbereich einer frühen Inspirationsphase eine hohe Sauerstoffkonzentration im Vergleich zu einer restlichen Inspirationszeit am Ende einer Beatmungsphase bereit gestellt wird. Es ist vorgesehen, dass die Sauerstoffkonzentration zwischen 20% und 100% liegt. Es ist vorgesehen, dass der Zeitbereich fest einstellbar ist. Es ist vorgesehen, dass der Zeitbereich frei wählbar ist. Es ist vorgesehen, dass die Berechnung des Zeitbereiches abhängig von physiologischen, gerätetechnischen und/oder beatmungsrelevanten Parameter erfolgt. Es ist vorgesehen, dass als physiologischer Parameter mindestens einer der folgenden Parameter genutzt wird: Hämoglobinwert, et CO2, sp02 oder weitere direkte oder indirekte Indikatoren für eine Oxygenierung des Blutes. Es ist vorgesehen, dass als gerätetechnischer Parameter mindestens einer der folgenden Parameter genutzt wird: Schlauchvolumen, Schlauchlänge, Volumen der gesamten Beatmungsstrecke, Volumen Geräteblockes, pneumatische Widerstände der Beatmungsgerätkomponenten sowie weitere zur Berechnung der richtigen Applikationszeitpunkte des Sauerstoffes notwendige technische Parameter. Es ist vorgesehen, dass als beatmungsrelevante Parameter mindestens einer der folgenden Parameter genutzt wird: Inspirationszeit, Inspirationsflow, Inspirationsdruck, Druck-, Flow- und Volumen-Kriterien, Inspirationszeit Ti, Exspirationszeit. Es ist vorgegeben, dass die Vorlaufzeit zum Auslösen der erhöhten Sauerstoffkonzentration des Gerätes über mindestens dem Flow und das Beatmungsstreckenvolumen berechnet wird.

## Patentansprüche

1. Verfahren zur Ermittlung der Konzentration mindestens eines Anteils eines Gasgemisches bestehend aus mindestens zwei Gasen mit bekannten Konzentrationen der jeweiligen Inhaltsstoffe unter Verwendung einer Vorrichtung mit einer Benutzerschnittstelle (11), einem mindestens eines der zu vermischenden Gase oder ein Gasgemisch zuführenden und an eine Gasquelle (13) angeschlossenen Schlauch (10'), einem Sauerstoff zuführenden und an eine Sauerstoffquelle angeschlossenen Sauerstoffschlauch (16), mit Mitteln (10") zum Ablassen der ausgeatmeten Luft, mit einem den Volumenstrom- und/oder Massenstrom mindestens eines der zu vermischenden Gase regelnden Regelventil (15, 18) und mit einer Steuerung (7), bei der die Vorrichtung über mindestens eine Erfassungseinrichtung für den Volumenstrom und/oder Massenstrom oder die Gaszusammensetzung mindestens eines der zu vermischenden Gase oder Gasgemische verfügt, und die Steuerung ausgebildet ist, den mindestens einen erfassten Volumenstromwert des mindestens einen zu vermischenden Gases oder Gasgemischs zu verarbeiten, und den Volumenstromwert mindestens eines der zu vermischenden Gase oder Gasgemische zu steuern und dem Volumenstrom eines ersten Gases, welches eine Transportleitung durchströmt, zumindest Sauerstoff als ein zweites Gas wenigstens zeitweise im Bereich einer Mischstelle zuzudosieren und die Volumenströme der zu vermischenden Gase sensorisch zu erfassen und die verschobenen Volumina der Gase in der jeweiligen Transportleitung zu berechnen, wobei die Berechnung der Mischkonzentration im Bereich der Mischstelle aus den berechneten Konzentrationen der verschobenen Volumina erfolgt, **dadurch gekennzeichnet, dass** entlang der Transportleitung an zumindest zwei Punkten die Verteilung der Konzentration von Sauerstoff im Gasgemisch berechnet wird.

2. Verfahren nach Anspruch 1, **dadurch gekennzeichnet, dass** eines der zu vermischenden Gase oder Gasgemische mit einem konstanten Volumenstrom und/oder Massenstrom zugeführt wird, und hierfür keine Erfassung des Volumenstroms und/oder Massenstroms durchgeführt wird.

3. Verfahren nach einem der Ansprüche 1 oder 2, **dadurch gekennzeichnet, dass** die CO2-Konzentration im Bereich unter der Benutzerschnittstelle berechnet wird.

4. Verfahren nach einem der Ansprüche 1 bis 3, **dadurch gekennzeichnet, dass** die Stickstoff-Konzentration im Bereich unter der Benutzerschnittstelle berechnet wird.

5. Verfahren nach einem der Ansprüche 1 bis 4, **dadurch gekennzeichnet, dass** im Falle einer Abweichung der berechneten Gaskonzentration von dem vorgegebenen Sollwert ein Alarm ausgelöst wird.

6. Verfahren nach einem der Ansprüche 1 bis 5, **dadurch gekennzeichnet, dass** zur Bereitstellung eines aus mindestens zwei Gasen bestehenden Atemgasgemisches eine Vorrichtung mit einer Benutzerschnittstelle (11), einem mindestens eines der zu vermischenden Gase oder ein Gasgemisch zuführenden und an eine Gasquelle (13) angeschlossenen Schlauch (10'), einem Sauerstoff zuführenden und an eine Sauerstoffquelle angeschlossenen Sauerstoffschlauch (16), mit Mitteln (10") zum Ablassen der ausgeatmeten Luft, mit einem den Volumenstrom- und/oder Massenstrom mindestens eines zu der vermischenden Gase regelnden Regelventil (15, 18) und mit einer Steuerung (7), verwendet wird, bei der die Vorrichtung über mindestens eine Erfassung des Volumenstroms und/oder Massenstromes oder der Gaszusammensetzung mindestens eines der zu vermischenden Gase oder Gasgemische verfügt, und die Steuerung den mindestens einen erfassten Volumenstrom- und/oder Massenstromwert des mindestens einen zu vermischenden Gases oder Gasgemischs verarbeitet, und den Volumenstrom und/oder Massenstrom mindestens eines der zu vermischenden Gase oder Gasgemische steuert.

7. Verfahren nach Anspruch 1, **dadurch gekennzeichnet, dass** eines der zu vermischenden Gase ein Gasgemisch ist.

8. Verfahren nach Anspruch 1, **dadurch gekennzeichnet, dass** die Erfassung des Volumenstroms und/oder Massenstroms sich auf mindestens ein zweites zu vermischendes Gas oder ein Gasgemisch erstreckt und die Steuerung (7) auch diese/n Messwert/e verarbeitet.

9. Verfahren nach Anspruch 1, **dadurch gekennzeichnet, dass** der Sauerstoffschlauch (16) im inneren des Luft zuführenden Schlauchs verlegt wird.

10. Verfahren nach Anspruch 1, **dadurch gekennzeichnet, dass** der Sauerstoffschlauch (16) außerhalb der Luft zuführenden Schlauchs verlegt wird.

11. Verfahren nach einem der vorhergehenden Ansprüche, **dadurch gekennzeichnet, dass** die Einleitstelle (4) des Sauerstoffs in den Luft zuführenden Schlauch (10') zwischen seinem Anschluss an der Benutzerschnittstelle (11) und seinem Anschluss an die Gasquelle positioniert ist.

12. Verfahren nach einem der vorhergehenden Ansprüche, **dadurch gekennzeichnet, dass** die Steuerung den Volumenstrom und/oder Massenstrom mindestens eines weiteren zu vermischenden Gases steuert.

13. Verfahren nach einem der vorhergehenden Ansprüche, **dadurch gekennzeichnet, dass** mindestens eines der zu vermischenden Gase oder Gasgemische mit einem konstanten Volumenstrom und/oder Massenstrom in den zugehörigen Schlauch eingeführt wird.

14. Verfahren nach einem der vorhergehenden Ansprüche, **dadurch gekennzeichnet, dass** als Sauerstoffquelle ein Druckbehälter mit vorkomprimiertem Sauerstoff verwendet wird.

15. Verfahren nach einem der vorhergehenden Ansprüche, **dadurch gekennzeichnet, dass** als Sauerstoffquelle ein Sauerstofferzeuger verwendet wird.

16. Verfahren nach einem der vorhergehenden Ansprüche, **dadurch gekennzeichnet, dass** die Erfassung des Volumenstrom und/oder Massenstrom mittels zumindest eines Durchflussmessgerätes erfolgt.

17. Verfahren nach einem der vorhergehenden Ansprüche, **dadurch gekennzeichnet, dass** die Regelung des Volumendurchflusses zumindest eines der zu vermischenden Stoffe mittels zumindest eines elektromechanisch angetriebenen Regelventils (15, 18) erfolgt.

18. Verfahren nach einem der vorhergehenden Ansprüche, **dadurch gekennzeichnet, dass** die Steuerung (7) über eine Schnittstelle verfügt, die eingehende Signale von einem oder mehreren Volumenstrom und/oder Massenstrom-Sensoren empfängt, und ausgehende Signale für ein oder mehrere Regelventile ausgibt.

19. Verfahren nach einem der vorhergehenden Ansprüche, **dadurch gekennzeichnet, dass** die Steuerung (7) mit einer Anzeigevorrichtung (19) versehen wird.

20. Verfahren nach einem der vorhergehenden Ansprüche, **dadurch gekennzeichnet, dass** die Steuerung (7) mit einer Eingabevorrichtung (20) versehen wird.

21. Verfahren nach einem der vorhergehenden Ansprüche, **dadurch gekennzeichnet, dass** die Benutzerschnittstelle als eine Atemmaske oder ein Endotrachealtubus oder ein Tracheostoma ausgeführt wird.

22. Verfahren nach einem der vorhergehenden Ansprüche, **dadurch gekennzeichnet, dass** die Gasquelle als ein Gebläse ausgeführt wird.

23. Verfahren nach einem der vorhergehenden Ansprüche, **dadurch gekennzeichnet, dass** die Gasquelle als ein vorkomprimierter Druckbehälter ausgeführt wird.

## Claims

1. Method for determining the concentration of at least one fraction of a gas mixture consisting of at least two gases with known concentrations of the respective contents using an apparatus having a user interface (11), a hose (10') which is connected to a gas source (13) and supplies at least one of the gases to be mixed or a gas mixture, an oxygen hose (16) which is connected to an oxygen source and supplies oxygen, having means (10'') for venting the exhaled air, having a regulator valve (15, 18) which regulates the volume flow and/or mass flow of at least one of the gases to be mixed, and having a control unit (7), in which the apparatus has at least one detection device for the volume flow and/or mass flow or the gas composition of at least one of the gases or gas mixtures to be mixed, and the control unit is designed to process the at least one detected volume flow value of the at least one gas or gas mixture to be mixed, and to control the volume flow value of at least one of the gases or gas mixtures to be mixed, and to meter, to the volume flow of a first gas flowing through a transport line, at least oxygen as a second gas at least temporarily in the region of a mixing point, and to detect by means of sensors the volume flows of the gases to be mixed, and to calculate the displaced volumes of the gases in the respective transport line, wherein the mixing concentration is calculated in the region of the mixing point using the calculated concentrations of the displaced volumes, **characterized in that** the distribution of the oxygen concentration in the gas mixture is calculated at at least two points along the transport line.

2. Method according to Claim 1, **characterized in that** one of the gases or gas mixtures to be mixed is supplied with a constant volume flow and/or mass flow, and for this no detection of the volume flow and/or mass flow is performed.

3. Method according to Claim 1 or 2, **characterized in that** the CO2 concentration is calculated in the region below the user interface.

4. Method according to one of Claims 1 to 3, **characterized in that** the nitrogen concentration is calculated in the region below the user interface.

5. Method according to one of Claims 1 to 4, **characterized in that** an alarm is triggered in the event of the calculated gas concentration deviating from the predefined setpoint value.

6. Method according to one of Claims 1 to 5, **characterized in that**, in order to provide a breathing gas mixture consisting of at least two gases, use is made of an apparatus having a user interface (11), a hose (10') which is connected to a gas source (13) and supplies at least one of the gases to be mixed or a gas mixture, an oxygen hose (16) which is connected to an oxygen source and supplies oxygen, having means (10'') for venting the exhaled air, having a regulator valve (15, 18) which regulates the volume flow and/or mass flow of at least one of the gases to be mixed, and having a control unit (7), in which the apparatus has at least one detection of the volume flow and/or mass flow or of the gas composition of at least one of the gases or gas mixtures to be mixed, and the control unit processes the at least one detected volume flow value and/or mass flow value of the at least one gas or gas mixture to be mixed, and controls the volume flow and/or mass flow of at least one of the gases or gas mixtures to be mixed.

7. Method according to Claim 1, **characterized in that** one of the gases to be mixed is a gas mixture.

8. Method according to Claim 1, **characterized in that** the detection of the volume flow and/or mass flow extends to at least one second gas to be mixed or a gas mixture, and the control unit (7) also processes this/these measurement value(s).

9. Method according to Claim 1, **characterized in that** the oxygen hose (16) is laid in the interior of the air-supplying hose.

10. Method according to Claim 1, **characterized in that** the oxygen hose (16) is laid outside the air-supplying hose.

11. Method according to one of the preceding claims, **characterized in that** the introduction point (4) of the oxygen into the air-supplying hose (10') is positioned between its connection to the user interface (11) and its connection to the gas source.

12. Method according to one of the preceding claims, **characterized in that** the control unit controls the volume flow and/or mass flow of at least one other gas to be mixed.

13. Method according to one of the preceding claims, **characterized in that** at least one of the gases or gas mixtures to be mixed is introduced into the appropriate hose with constant volume flow and/or mass flow.

14. Method according to one of the preceding claims, **characterized in that** a pressure vessel with precompressed oxygen is used as the oxygen source.

15. Method according to one of the preceding claims, **characterized in that** an oxygen generator is used as the oxygen source.

16. Method according to one of the preceding claims, **characterized in that** the volume flow and/or mass flow is detected using at least one through-flow measurement device.

17. Method according to one of the preceding claims, **characterized in that** the volume through-flow of at least one of the substances to be mixed is regulated using at least one electro-mechanically driven regulator valve (15, 18).

18. Method according to one of the preceding claims, **characterized in that** the control unit (7) has an interface which receives incoming signals from one or more volume flow and/or mass flow sensors, and emits outgoing signals for one or more regulator valves.

19. Method according to one of the preceding claims, **characterized in that** the control unit (7) is provided with a display device (19).

20. Method according to one of the preceding claims, **characterized in that** the control unit (7) is provided with an input device (20).

21. Method according to one of the preceding claims, **characterized in that** the user interface is in the form of a breathing mask or an endotracheal tube or a tracheostomy.

22. Method according to one of the preceding claims, **characterized in that** the gas source is in the form of a ventilator.

23. Method according to one of the preceding claims, **characterized in that** the gas source is in the form of a previously pressurized pressure vessel.

## Revendications

1. Procédé pour déterminer la concentration d'au moins un composant d'un mélange gazeux se composant d'au moins deux gaz avec des concentrations connues des substances contenues respectives avec utilisation d'un dispositif muni d'une interface d'utilisateur (11), d'un tuyau flexible (10') amenant au moins un des gaz à mélanger ou un mélange gazeux et raccordé à une source de gaz (13), d'un tuyau flexible à oxygène (16) amenant de l'oxygène et raccordé à une source d'oxygène, avec des moyens (10") pour rejeter l'air expiré, avec une soupape de réglage (15, 18) réglant le débit volumétrique et/ou le débit massique d'au moins un des gaz à mélanger et avec une commande (7), dans laquelle le dispositif comprend au moins un dispositif de détection pour le débit volumétrique et/ou le débit massique ou pour la composition de gaz d'au moins un des gaz ou mélanges gazeux à mélanger, et la commande est conçue pour traiter ladite au moins une valeur de débit volumétrique détectée dudit au moins un gaz ou mélange gazeux à mélanger, et pour commander la valeur de débit volumétrique d'au moins un des gaz ou mélanges gazeux à mélanger et pour ajouter par doses au courant volumétrique d'un premier gaz, qui s'écoule dans une conduite de transport, au moins de l'oxygène comme deuxième gaz au moins temporairement dans la région d'une zone de mélange et pour détecter par des capteurs les débits volumétriques des gaz à mélanger et pour calculer les volumes déplacés des gaz dans la conduite de transport respective, dans lequel on effectue le calcul de la concentration du mélange dans la région de la zone de mélange à partir des concentrations calculées des volumes déplacés, **caractérisé en ce que** l'on calcule la distribution de la concentration d'oxygène dans le mélange gazeux en au moins deux points le long de la conduite de transport.

2. Procédé selon la revendication 1, **caractérisé en ce qu'**un des gaz ou mélanges gazeux à mélanger est fourni avec un débit volumétrique et/ou un débit massique constant, et on n'effectue à cet effet aucune détection du débit volumétrique et/ou du débit massique.

3. Procédé selon une des revendications 1 ou 2, **caractérisé en ce que** l'on calcule la concentration de CO₂ dans la région située sous l'interface d'utilisateur.

4. Procédé selon l'une quelconque des revendications 1 à 3, **caractérisé en ce que** l'on calcule la concentration d'azote dans la région située sous l'interface d'utilisateur.

5. Procédé selon l'une quelconque des revendications 1 à 4, **caractérisé en ce qu'**une alarme est déclenchée dans le cas d'un écart de la concentration de gaz calculée par rapport à la valeur de consigne prédéterminée.

6. Procédé selon l'une quelconque des revendications 1 à 5, **caractérisé en ce que** pour la fourniture d'un mélange gazeux pour la respiration composé d'au moins deux gaz, on utilise un dispositif muni d'une interface d'utilisateur (11), d'un tuyau flexible (10') amenant au moins un des gaz à mélanger ou un mélange gazeux et raccordé à une source de gaz (13), d'un tuyau flexible à oxygène (16) amenant de l'oxygène et raccordé à une source d'oxygène, avec des moyens (10") pour rejeter l'air expiré, avec une soupape de réglage (15, 18) réglant le débit volumétrique et/ou le débit massique d'au moins un des gaz à mélanger et avec une commande (7), dans laquelle le dispositif comprend au moins une détection du débit volumétrique et/ou du débit massique ou de la composition de gaz d'au moins un des gaz ou mélanges gazeux à mélanger, et la commande traite ladite au moins une valeur de débit volumétrique et/ou de débit massique détectée dudit au moins un gaz ou mélange gazeux à mélanger, et commande le débit volumétrique et/ou le débit massique d'au moins un des gaz ou mélanges gazeux à mélanger.

7. Procédé selon la revendication 1, **caractérisé en ce qu'**un des gaz à mélanger est un mélange gazeux.

8. Procédé selon la revendication 1, **caractérisé en ce que** la détection du débit volumétrique et/ou du débit massique s'étend sur au moins un deuxième gaz ou mélange gazeux à mélanger et la commande (7) traite également cette/ces valeur(s) de mesure.

9. Procédé selon la revendication 1, **caractérisé en ce que** l'on pose le tuyau flexible à oxygène (16) à l'intérieur du tuyau flexible amenant de l'air.

10. Procédé selon la revendication 1, **caractérisé en ce que** l'on pose le tuyau flexible à oxygène (16) à l'extérieur du tuyau flexible amenant de l'air.

11. Procédé selon l'une quelconque des revendications précédentes, **caractérisé en ce que** le point d'introduction (4) de l'oxygène dans le tuyau flexible (10') amenant de l'air est positionné entre son raccordement à l'interface d'utilisateur (11) et son raccordement à la source de gaz.

12. Procédé selon l'une quelconque des revendications précédentes, **caractérisé en ce que** la commande commande le débit volumétrique et/ou le débit massique d'au moins un autre gaz à mélanger.

13. Procédé selon l'une quelconque des revendications précédentes, **caractérisé en ce que** l'on introduit au moins un des gaz ou mélanges gazeux à mélanger avec un débit volumétrique et/ou un débit massique constant dans le tuyau flexible respectif.

14. Procédé selon l'une quelconque des revendications précédentes, **caractérisé en ce que** l'on utilise comme source d'oxygène un réservoir sous pression contenant de l'oxygène pré-comprimé.

15. Procédé selon l'une quelconque des revendications précédentes, **caractérisé en ce que** l'on utilise comme source d'oxygène un générateur d'oxygène.

16. Procédé selon l'une quelconque des revendications précédentes, **caractérisé en ce que** l'on effectue la détection du débit volumétrique et/ou du débit massique au moyen d'au moins un appareil de mesure de débit.

17. Procédé selon l'une quelconque des revendications précédentes, **caractérisé en ce que** l'on effectue la régulation du débit volumétrique d'au moins une des substances à mélanger au moyen d'au moins une soupape de réglage (15, 18) à commande électromécanique.

18. Procédé selon l'une quelconque des revendications précédentes, **caractérisé en ce que** la commande (7) comporte une interface, qui reçoit des signaux entrants en provenance d'un ou de plusieurs capteur(s) de débit volumétrique et/ou de débit massique et envoie des signaux sortants pour une ou plusieurs soupape(s) de réglage.

19. Procédé selon l'une quelconque des revendications précédentes, **caractérisé en ce que** la commande (7) est dotée d'un dispositif d'affichage (19).

20. Procédé selon l'une quelconque des revendications précédentes, **caractérisé en ce que** la commande (7) est dotée d'un dispositif d'entrée (20).

21. Procédé selon l'une quelconque des revendications précédentes, **caractérisé en ce que** l'interface d'utilisateur est réalisée sous la forme d'un masque respiratoire ou d'une sonde endotrachéale ou d'un trachéostome.

22. Procédé selon l'une quelconque des revendications précédentes, **caractérisé en ce que** la source de gaz est réalisée sous la forme d'un ventilateur.

23. Procédé selon l'une quelconque des revendications précédentes, **caractérisé en ce que** la source de gaz est réalisée sous la forme d'un réservoir sous pression pré-comprimé.
